# EUROPEAN PATENT APPLICATION

(11) **EP 4 796 625 A1**
(43) Date of publication of application: **26.08.2026**
(21) Application number: 24897020.4
(22) Date of filing: 29.08.2024
(51) Int. Cl.: C12M 1/26

(54) **WORK DEVICE HAVING OPENING/CLOSING DOOR**

(30) Priority: 29.11.2023 JP 2023201419
(71) Applicant: Yamaha Hatsudoki Kabushiki Kaisha, Iwata-shi, Shizuoka 438-8501 (JP)
(72) Inventor: IZUME, Yohei, Iwata-shi, Shizuoka 438-8501 (JP)
(74) Representative: Müller-Boré & Partner Patentanwälte PartG mbB
(86) International application number: PCT/JP2024/030880
(87) International publication number: WO 2025/115329

(57) **Abstract**

A work device includes a housing including an opening/closing door, a work unit, a motor device, a first magnetic coupling member, a second magnetic coupling member, and a mechanical mechanism. The work unit is housed in the housing and performs predetermined work on an object disposed inside the housing. The motor device includes a motor shaft that outputs a driving force for operating the opening/closing door. The first magnetic coupling member is attached to the motor shaft. The second magnetic coupling member is magnetically coupled to the first magnetic coupling member. The mechanical mechanism mechanically connects the second magnetic coupling member and the opening/closing door, and transmits, to the opening/closing door, a driving force of the motor shaft applied from the first magnetic coupling member to the second magnetic coupling member by the coupling.

## Description

### Technical Field

The present invention relates to a work device having a housing and an opening/closing door attached to the housing.

### Background Art

Various work devices each including a housing that houses a work unit therein often include an opening/closing door that opens and closes an opening or the like for accessing the work unit. For example, an opening/closing door is provided also in a cell moving device that moves cells from a culture container to an inspection container using a head unit including a head to which a suction tip is attached (see, e.g., Patent Literature 1). The opening/closing door opens and closes an opening for accessing a container or a head unit disposed in a housing of the cell moving device. The opening/closing door prevents dust from entering the housing through the opening and prevents a worker from accessing the work unit in operation.

The opening/closing door may be an automatic door. In a case of an automatic door, it is necessary to provide a work device with a safety mechanism for preventing a phenomenon such as a finger of a worker being caught. The safety mechanism includes at least a sensor that detects abnormal operation of the opening/closing door due to catching of a foreign substance or the like, and a mechanism that reversely moves the opening/closing door at the time of the abnormal operation. As the sensor, a plurality of types of sensors for monitoring operation of the opening/closing door and the like from multiple sides are required. Furthermore, a backup mechanism on the assumption of a failure of these sensors is also required. Therefore, there has been a problem that a structure of the work device becomes complicated.

### Citation List

### Patent Literature

Patent Literature 1: Japanese Patent No. 6262254

### Summary of Invention

An object of the present invention is to provide a work device having an opening/closing door and enabling avoidance of complication of a structure while securing safety.

A work device according to one aspect of the present invention includes: a housing; a work unit that is housed in the housing and performs predetermined work on an object disposed inside the housing; an opening/closing door attached to the housing; a motor device including a motor shaft that outputs a driving force for operating the opening/closing door; a first magnetic coupling member attached to the motor shaft; a second magnetic coupling member magnetically coupled to the first magnetic coupling member; and a mechanical mechanism that mechanically connects the second magnetic coupling member and the opening/closing door, and transmits, to the opening/closing door, a driving force of the motor shaft applied from the first magnetic coupling member to the second magnetic coupling member by the coupling.

### Brief Description of Drawings

FIG. 1A is a perspective view illustrating an appearance of a cell moving device which is an example of a work device according to the present invention, and is a view illustrating a state in which an opening/closing door is opened.
FIG. 1B is a perspective view illustrating a state in which the opening/closing door is closed in the cell moving device.
FIG. 2 is a perspective view illustrating a work unit included in the cell moving device illustrated in FIGS. 1A and 1B.
FIG. 3 is a perspective view of an automatic door device included in the cell moving device.
FIG. 4 is a perspective view of the automatic door device in which a perspective direction is reversed from that of FIG. 3.
FIG. 5 is a perspective view of a magnetic coupling part.
FIG. 6 is a perspective view of a magnetic coupling part according to a modification.
FIG. 7A is a cross-sectional view illustrating an example of a guide member of the opening/closing door.
FIG. 7B is a cross-sectional view illustrating an example of the guide member of the opening/closing door.
FIG. 8A is a view illustrating operation of the automatic door device, and is a front view illustrating a state in which the opening/closing door is closed.
FIG. 8B is a side view of the automatic door device of FIG. 8A.
FIG. 9A is a view illustrating the operation of the automatic door device, and is a front view illustrating a state in which the opening/closing door is opened.
FIG. 9B is a side view of the automatic door device of FIG. 9A.
FIG. 10 is a cross-sectional view illustrating a modification of the opening/closing door and a guide member thereof.
FIG. 11A is a perspective view illustrating a modification of the automatic door device.
FIG. 11B is a schematic view of the magnetic coupling part.

### Description of Embodiments

In the following, embodiments of the present invention will be described in detail with reference to the accompanying drawings. The present invention can be applied to various work devices each including a housing, a work unit that is housed in the housing and performs predetermined work on an object disposed inside the housing, and an opening/closing door attached to the housing. For example, in a surface mounting line for mounting a component on a substrate, the present invention can be applied to a surface mounting machine having an opening/closing door, a printing machine, a bond coating device, and the like. In addition, the present invention can be applied to a work device that performs various works such as assembly, joining, transfer, inspection, cleaning, heating, drying, welding, and laser irradiation/machining inside a housing. Embodiments to be described below exemplify, as a work device, a cell moving device in which a first container serving as a movement source of a cell and a second container serving as a movement destination of a cell are disposed in a housing, and cells are picked from the first container and transferred to the second container.

### [Configuration of Cell Moving Device]

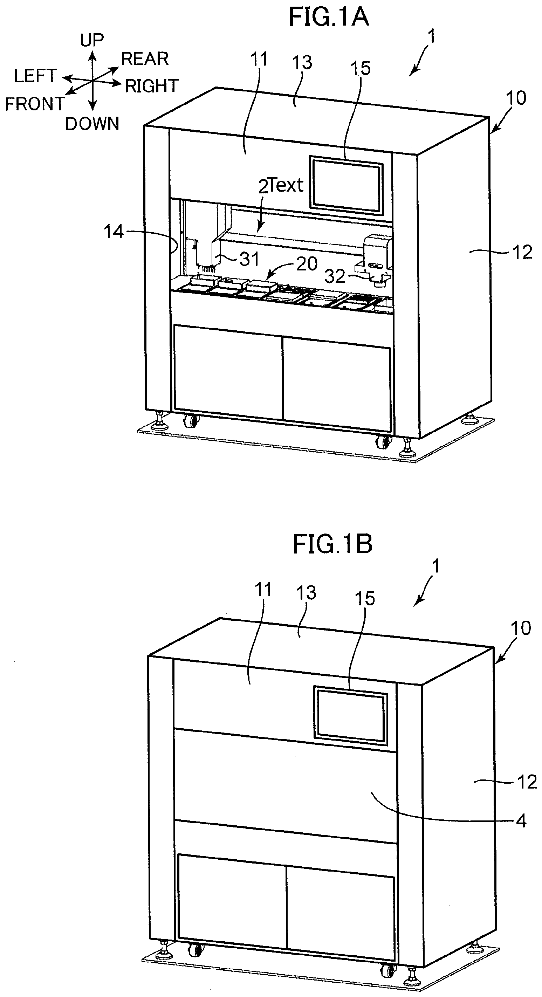

FIGS. 1A and 1B are perspective views illustrating an appearance of a cell moving device 1 according to the present embodiment. The cell moving device 1 includes a housing 10, a work unit 2 housed in the housing 10, and an opening/closing door 4 attached to the housing 10. FIG. 1A illustrates a state in which the opening/closing door 4 is opened, and FIG. 1B illustrates a state in which the opening/closing door 4 is closed. Note that, while in FIG. 1A and other drawings, directions are indicated as up-down, left-right, and front-rear, these are examples of directions and do not limit the directions at all.

The housing 10 has a rectangular parallelepiped shape that is long in left and right, and includes a front plate 11 located in front of the work unit 2, a pair of left and right side plates 12 covering the left and right of the work unit 2, and a top plate 13 covering over the work unit 2. The front plate 11 is provided with a rectangular opening 14 near the middle in the up-down direction. The opening 14 is an opening that enables access to the work unit 2 housed in the housing 10. A worker can perform necessary work such as setting of cells, supplement of consumables, or maintenance on the work unit 2 through the opening 14. The opening 14 is opened and closed by the opening/closing door 4.

The work unit 2 performs predetermined work on an object disposed inside the housing 10. In the present embodiment, the object is a cell, the predetermined work is mainly movement of the cell, and works such as cell culture, observation, and reagent administration can also be performed. When the work unit 2 is in an operating state, the opening/closing door 4 is basically in a closed state. A monitor 15 is attached near an upper end of the front plate 11. An operating status of the work unit 2, a state of cells, and the like can be confirmed through the monitor 15.

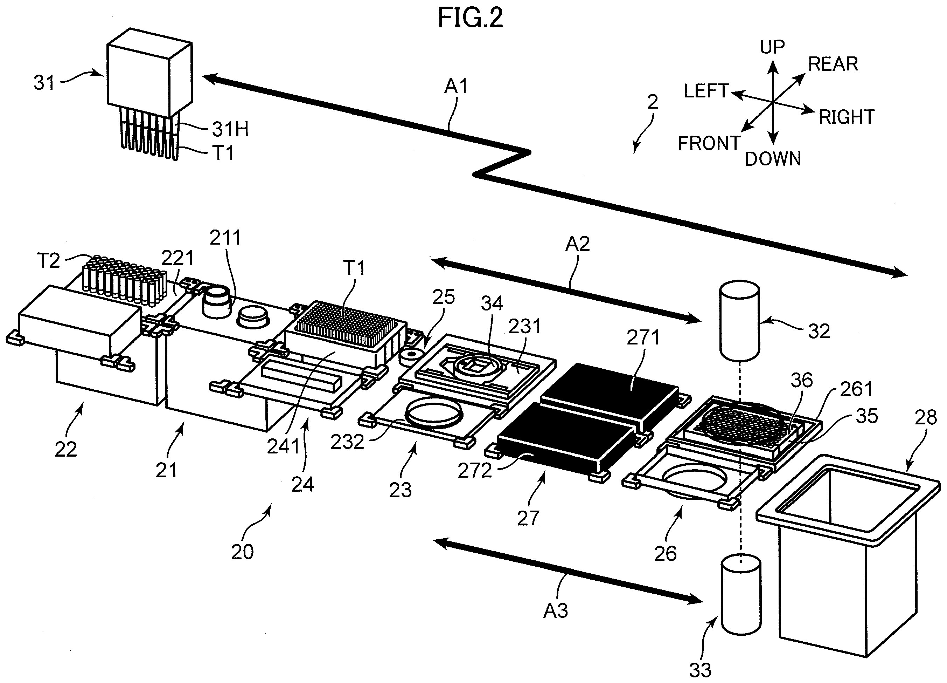

FIG. 2 is a perspective view of the work unit 2. The work unit 2 includes a cell movement line 20, a head unit 31, a lighting unit 32, and an imaging unit 33. The head unit 31 includes a plurality of heads 31H arranged in a line in the left-right direction. The head 31H is provided with a mechanism that generates a negative pressure or a positive pressure. A suction tip T1 for sucking cells is attached to a lower end of the head 31H. When a positive pressure or a negative pressure is generated in the head 31H, the suction tip T1 sucks or discharges the cells. As indicated by an arrow A1, the head unit 31 is movable in the left-right direction and the front-rear direction, and can move along a predetermined movement path on the cell movement line 20. The head 31H can move in the up-down direction.

In the work unit 2 in the housing 10, a dish 34 as a first container serving as a movement source of a cell and a microplate 35 as a second container serving as a movement destination of a cell are disposed. The cell movement line 20 is configured with elements arranged in the left-right direction, the elements being necessary for the head unit 31 to perform a series of cell moving steps of picking a cell stored in the dish 34 and moving the cell to the microplate 35. The cell movement line 20 includes a dispensing tip stock part 22, a cell stock part 21, a tip stock part 24, a tip imaging part 25, a cell sorting part 23, a black cover placement part 27, a cell transfer part 26, and a tip discarding part 28 that are arranged in a line from left to right.

The cell stock part 21 includes a tube 211 that stores a cell culture solution in which a large amount of cells are dispersed, the tube serving as a dispensing source. The tube 211 is made of a cylindrical container with an open upper surface. The dispensing tip stock part 22 is an area for keeping a plurality of dispensing tips T2. The dispensing tip stock part 22 includes a holder 221 that holds the dispensing tips T2 lined in a matrix in an erected state. The dispensing tip T2 is also attached to the head 31H. The dispensing tip T2 is used to suck the cell culture solution from the tube 211 and discharge the cell culture solution to the dish 34.

The cell sorting part 23 is a part for sorting desired cells from the cell culture solution containing a large number of cells. The cell sorting part 23 includes a holding table 231 that positions and holds the dish 34 that stores the cell culture solution, and a table lid member 232 that covers an upper surface of the dish 34. The dish 34 includes a plurality of recesses for carrying cells on the upper surface side. An image of the cell in a state of being carried in the recess is captured by the imaging unit 33 under lighting of the lighting unit 32. As a result, a position of a target cell to be sucked is specified.

The tip stock part 24 includes a holding box 241 that holds a large number of the suction tips T1 arranged in a matrix. The suction tip T1 can be attached to and detached from the head 31H. The suction tip T1 is held in the holding box 241 in a state of being easily attached to the head 31H moving in the up-down direction. The suction tip T1 has a function of sucking a cell carried on the dish 34, transporting the cell as the head unit 31 moves, and discharging the cell to the cell transfer part 26.

The tip imaging part 25 is an area that captures an image of the suction tip T1 attached to the head 31H. The imaging is performed by the imaging unit 33. An XYZ coordinate position of a distal end suction port of the suction tip T1 is obtained on the basis of the image of the suction tip T1 and focal position information at the time of imaging. A correction value is derived from a difference between the coordinate position and a predetermined reference position, and is used as a correction value at the time of movement control of the head 31H.

The cell transfer part 26 is an area where the microplate 35, which is a movement destination of the cell picked from the dish 34 of the cell sorting part 23, is disposed. The microplate 35 is held by a holding table 261. The microplate 35 is a plate in which a large number of small wells 36 having open upper surfaces are arranged in a matrix. The cells held in the suction tip T1 are discharged to these wells 36. Note that the microplate 35 may be placed on the cell sorting part 23.

The black cover placement part 27 is a part on which a first black cover 271 covering the cell transfer part 26 and a second black cover 272 covering the cell sorting part 23 are placed. The first and second black covers 271 and 272 are used to image cell aggregates carried on the dish 34 or the microplate 35 in a light-shielded state for fluorescence observation of cell. The tip discarding part 28 is an area where the suction tip T1 and the dispensing tip T2 after use, which have finished suction and discharging operations of the cells, are discarded.

The lighting unit 32 is movably disposed above the cell movement line 20 to exclusively light the cell sorting part 23 and the cell transfer part 26 from above. The lighting is used as transmitted lighting when the cell held in the cell sorting part 23 or the cell transfer part 26 is imaged by the imaging unit 33. The lighting unit 32 is movable in the left-right direction and the front-rear direction.

The imaging unit 33 images the cells held by the cell sorting part 23 and the cell transfer part 26 from below. The imaging unit 33 is movable in the left-right and front-rear directions below the cell movement line 20. The imaging unit 33 is also used to observe a state of attachment of the suction tip T1 to the head 31H at the tip imaging part 25.

An example of work using a cell as an object in the work unit 2 is as follows. The head 31H attached with the dispensing tip T2 is moved to the cell stock part 21, and the cell culture solution containing the cells is sucked from the tube 211 into the dispensing tip T2. The head 31H is moved onto the cell sorting part 23 to cause the cell culture solution in the dispensing tip T2 to be discharged to the dish 34. The dispensing tip T2 attached to the head 31H is discarded by the tip discarding part 28, and the suction tip T1 is attached to the head 31H at the tip stock part 24. After a distal end of the suction tip T1 is imaged by the tip imaging part 25, the head 31H is moved onto the cell sorting part 23, and the target cells stored in the dish 34 are sucked into the suction tip T1. Thereafter, the head 31H is moved onto the cell transfer part 26 to cause the cells in the suction tip T1 to be discharged to the microplate 65. The used suction tip T1 is discarded to the tip discarding part 28.

### [Configuration of Automatic Door Device]

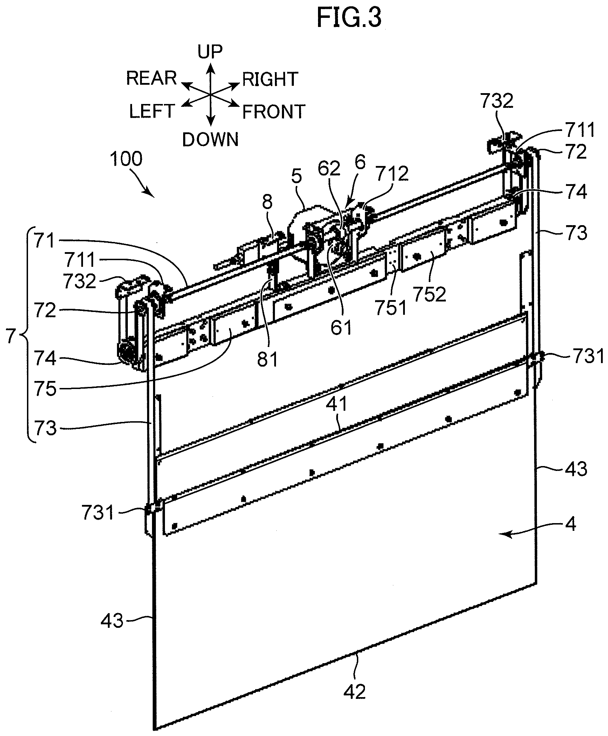

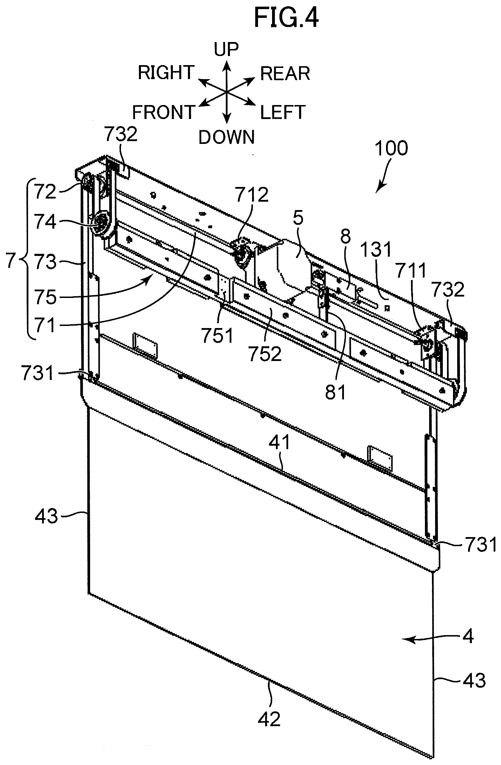

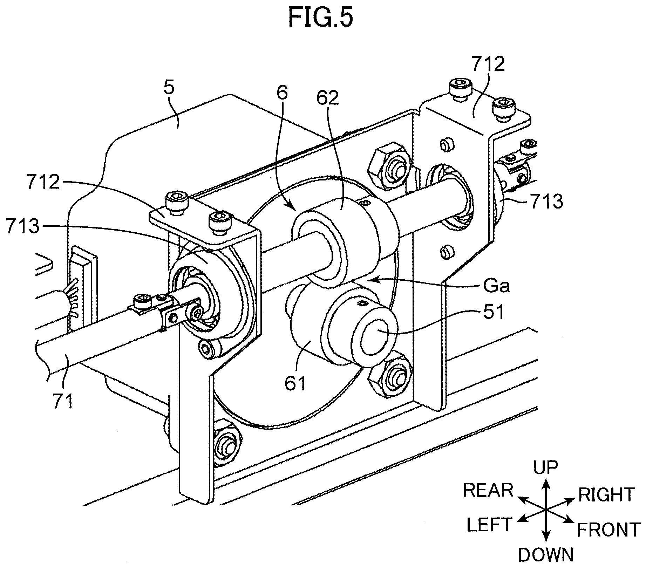

Next, an automatic door device 100 that automatically opens and closes the opening/closing door 4 will be described. FIGS. 3 and 4 are perspective views of the automatic door device 100 included in the cell moving device 1. The automatic door device 100 includes the opening/closing door 4, a motor device 5, a magnetic coupling part 6, a mechanical mechanism 7, and a sensor 8. FIG. 5 is an enlarged perspective view illustrating arrangement positions of the motor device 5 and the magnetic coupling part 6.

The opening/closing door 4 is formed of a flat plate having a rectangular shape in a plan view and having a size larger than the opening 14 of the housing 10, and moves up and down to open and close the opening 14. The motor device 5 is a drive source that opens and closes the opening/closing door 4. The motor device 5 includes a motor shaft 51 that outputs a driving force for operating the opening/closing door 4. As the motor device 5, a DC or AC motor can be used.

The magnetic coupling part 6 is a mechanism part that transmits the driving force in a non-contact manner using an attractive force and a repulsive force between magnets. The magnetic coupling part 6 includes a first magnetic coupling member 61 and a second magnetic coupling member 62 that are magnetically coupled to each other. The first magnetic coupling member 61 and the second magnetic coupling member 62 are both cylindrical bodies through which a shaft body can be inserted at the center. The cylindrical body is formed of sectored S-pole magnets and N-pole magnets alternately arranged in a circumferential direction. The first magnetic coupling member 61 is attached to the motor shaft 51. The second magnetic coupling member 62 is attached to a shaft 71 to be described later. The motor shaft 51 and the shaft 71 have a relationship of being arranged orthogonal to each other. That is, an axis (the motor shaft 51) to which the first magnetic coupling member 61 is attached extends in the front-rear direction, and an axis (the shaft 71) to which the second magnetic coupling member 62 is attached extends in the left-right direction, and they are orthogonal to each other.

The first magnetic coupling member 61 and the second magnetic coupling member 62 are not in mechanical contact with each other, and are disposed so as to face each other with a gap Ga having a predetermined width interposed therebetween. Although the gap Ga is interposed, when the first magnetic coupling member 61 rotates about the axis by the operation of the motor shaft 51, a rotational force is transmitted on the basis of a magnetic coupling force, and the second magnetic coupling member 62 also rotates about the axis. However, when a rotational load of the second magnetic coupling member 62 becomes larger than the magnetic coupling force, the rotational force will not be transmitted, so that the first magnetic coupling member 61 idles. The magnetic coupling force can be adjusted by the width of the gap Ga. The width of the gap Ga is set on the assumption that the magnetic coupling part 6 exhibits a torque limiter function when the opening/closing door 4 catches a finger of a worker.

The mechanical mechanism 7 mechanically connects the second magnetic coupling member 62 and the opening/closing door 4, and transmits, to the opening/closing door 4, a rotational driving force of the motor shaft 51 applied from the first magnetic coupling member 61 to the second magnetic coupling member 62 by the magnetic coupling. The mechanical mechanism 7 includes the shaft 71, a belt gear 72, a belt 73, a moving pulley 74, and a weight unit 75.

The shaft 71 is a cylindrical body extending left and right, and is disposed near the top plate 13 of the housing 10. The second magnetic coupling member 62 is attached to an intermediate position in an axial direction of the shaft 71. When the rotational driving force of the motor shaft 51 is applied from the first magnetic coupling member 61 to the second magnetic coupling member 62, the shaft 71 rotates about the axis. A left end and a right end of the shaft 71 are rotatably supported by an end shaft support part 711. A vicinity of the center of the shaft 71 is rotatably supported by a pair of intermediate shaft support parts 712 disposed so as to sandwich the second magnetic coupling member 62 in the axial direction. A bearing 713 through which the shaft 71 is inserted is attached to the intermediate shaft support part 712. The end shaft support part 711 and the intermediate shaft support part 712 are fixed to a top plate frame 131 illustrated in FIG. 4.

The belt gear 72 is attached to each of the left end (one end) and the right end (the other end) of the shaft 71. The belt gear 72 is an engaging wheel having a plurality of engaging teeth provided in a protruding manner on a peripheral surface thereof. The belt 73 suspends and supports the opening/closing door 4, and is disposed as a left and right pair. The pair of belts 73 is a so-called toothed belt, and is engaged with the shaft 71 via the belt gear 72. On one surface of the belt gear 72, a large number of ribs meshing with the engaging teeth of the belt gear 72 are provided in a protruding manner at a constant pitch. The left belt 73 (first belt) meshes with the belt gear 72 at the left end of the shaft 71, and the right belt 73 (second belt) meshes with the belt gear 72 at the right end. In this manner, the two belts 73 are attached to the left end and the right end of the shaft 71. A pulley having high friction may be used instead of the belt gear 72, a wire stretched around the pulley may be used instead of the belt 73, and the opening/closing door 4 may be suspended and supported by the wire.

The left belt 73 is disposed at a position corresponding to a left side end edge 43 of the opening/closing door 4, and the right belt 73 is disposed at a position corresponding to a right side end edge 43. A lower end of each belt 73 is a suspension support part 731. The suspension support part 731 is connected to the side end edge 43 near an upper end edge 41 of the opening/closing door 4, and suspends and supports the opening/closing door 4. A terminal fixing part 732 is attached to an upper end of each belt 73. As illustrated in FIG. 4, the terminal fixing part 732 is fixed to the top plate frame 131.

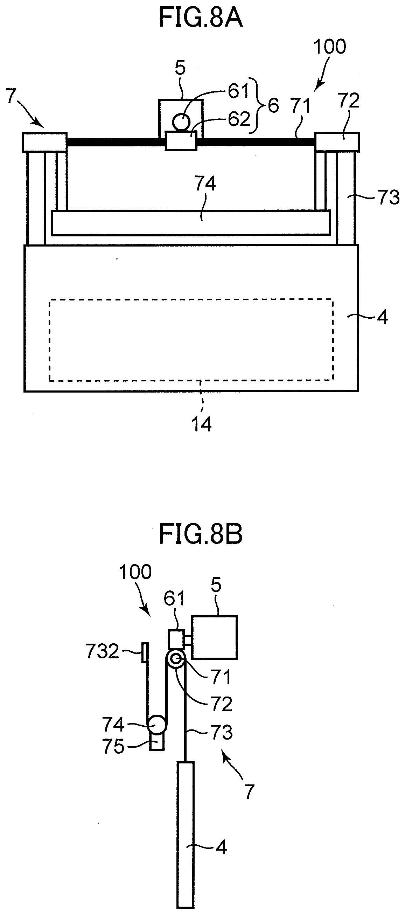

The belt 73 is stretched around the moving pulley 74, and the moving pulley 74 moves in the up-down direction in conjunction with up-down movement of the opening/closing door 4. A position of the moving pulley 74 illustrated in FIGS. 3 and 4 is a position when the opening/closing door 4 is lowered, i.e., a position when the opening 14 is closed. Operation of the opening/closing door 4 will be described with reference to FIGS. 8A to 9B. When viewed from the suspension support part 731, the belt 73 is stretched so as to be folded downward by the belt gear 72 and then folded upward by the moving pulley 74 to reach the terminal fixing part 732 at the upper end. The weight unit 75 is attached to the moving pulley 74.

The weight unit 75 includes a base material plate 751 and a weight member 752. The base material plate 751 is a flat plate having a left-right width equivalent to that of the opening/closing door 4. The moving pulley 74 is rotatably supported at left and right ends of the base material plate 751. The weight member 752 is a plate member having a predetermined weight and is held by the base material plate 751. A weight of the weight unit 75 can be appropriately set according to a capability of the motor device 5.

The sensor 8 detects an open/closed state of the opening/closing door 4. In the present embodiment, a limit switch type sensor 8 is adopted. A main body of the sensor 8 is attached to the top plate frame 131. On the other hand, a dog 81 for operating an actuator of the main body of the sensor 8 is provided in a protruding manner on an upper surface of the weight unit 75. The weight unit 75 rises in conjunction with the moving pulley 74, and the dog 81 interferes with the actuator, whereby a position of the opening/closing door 4 is detected.

The above embodiment shows the example in which the paired left and right belts 73 are engaged with the left end and the right end of the shaft 71. When the opening/closing door 4 has a small size, the shaft 71 and the opening/closing door 4 may be connected by one belt 73. Conversely, when the opening/closing door 4 is large, the shaft 71 and the opening/closing door 4 may be connected by three or more belts 73. Alternatively, the pair of left and right belts 73 may be engaged with the shaft 71 not at the end of the shaft 71 but at a position close to the center from the end. However, when the pair of belts 73 is engaged with the left end and the right end of the shaft 71 as in the present embodiment, the rectangular opening/closing door can be stably supported and stably moved up and down.

The above embodiment also shows the example in which the magnetic coupling part 6 is disposed at the intermediate position of the shaft 71. Alternatively, the magnetic coupling part 6 may be disposed at a position biased leftward or rightward from the intermediate position of the shaft 71. However, according to the present embodiment, the left and right belts 73 are respectively engaged with the opposite ends of the shaft, and the magnetic coupling part 6 to which the driving force is applied is disposed at the center of the shaft 71. Therefore, well-balanced power transmission can be realized.

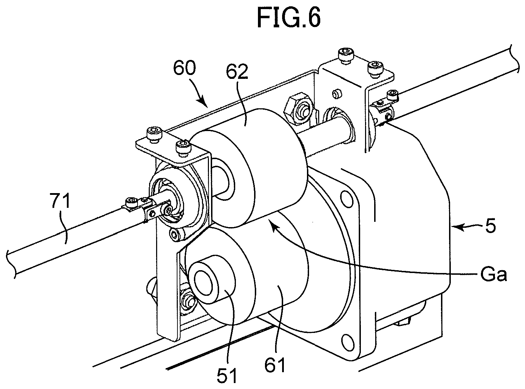

Furthermore, an installation mode of the magnetic coupling part 6 is not limited to the example of FIG. 5. FIG. 6 is a perspective view illustrating a magnetic coupling part 60 according to a modification. In the magnetic coupling part 60, as in the example of FIG. 5, the first magnetic coupling member 61 and the second magnetic coupling member 62 face each other with the gap Ga interposed therebetween. A difference from the example of FIG. 5 is that an axis of the first magnetic coupling member 61 and an axis of the second magnetic coupling member 62 are in the same direction.

In the example of FIG. 6, the motor shaft 51 and the shaft 71 are disposed to extend in the same direction. Therefore, the first magnetic coupling member 61 attached to the motor shaft 51 and the second magnetic coupling member 62 attached to the shaft 71 are oriented in the same direction. Such magnetic coupling part 60 can also transmit the rotational driving force of the motor shaft 51 to the shaft 71.

However, disposing the first magnetic coupling member 61 and the second magnetic coupling member 62 orthogonal to each other as illustrated in FIG. 5 has an advantage that a housability in the housing 10 can be improved. In general, the motor device 5 is larger in size than the shaft 71. For this reason, in the arrangement of FIG. 6, in order to house a part where the motor device 5 protrudes more than the shaft 71, it is necessary to make the housing 10 large or irregular so as to bulge forward. According to the arrangement of FIG. 5, since the motor device 5 can be disposed on the back side of the front plate 11, the housability of the motor device 5 in the housing 10 can be improved.

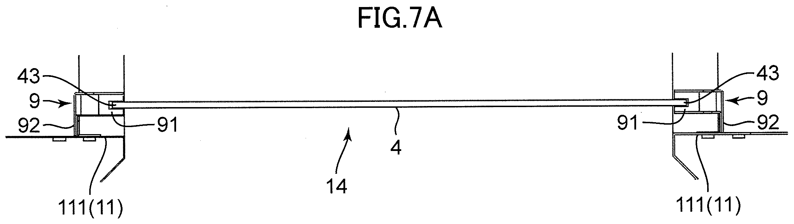

Since the opening/closing door 4 moves up and down while being suspended and supported by the belt 73, the opening/closing door can swing. Therefore, it is desirable to include a guide member that guides the movement of the opening/closing door 4 in the up-down direction. FIG. 7A is a cross-sectional view illustrating an example of a guide member 9 of the opening/closing door 4. The guide member 9 is disposed in the housing 10 at a position corresponding to the side end edge 43 (side) of the opening/closing door 4.

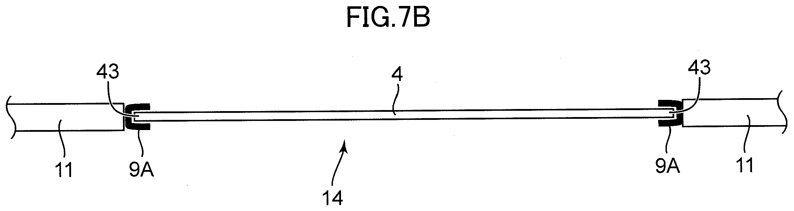

The front plate 11 includes an opening edge part 111 that defines the opening 14 around the opening 14. The guide member 9 includes a resin rail 91 extending in the up-down direction and a bracket 92. The resin rail 91 is a part that comes into contact with the opening/closing door 4, and has a U-shaped cross section so as to be able to receive the side end edge 43. That is, a part of the guide member 9 in contact with the opening/closing door 4 is made of resin. The bracket 92 is a metal fitting that holds the resin rail 91 and fixes the resin rail 91 to the opening edge part 111. Since the side end edge 43 is guided by the guide member 9 when the opening/closing door 4 moves in the up-down direction, the opening and closing operation of the opening/closing door 4 can be stabilized. As illustrated in FIG. 7B, the bracket 92 may be omitted, and a resin rail 9A may be directly fixed to the opening edge of the opening 14.

### [Operation of Automatic Door Device]

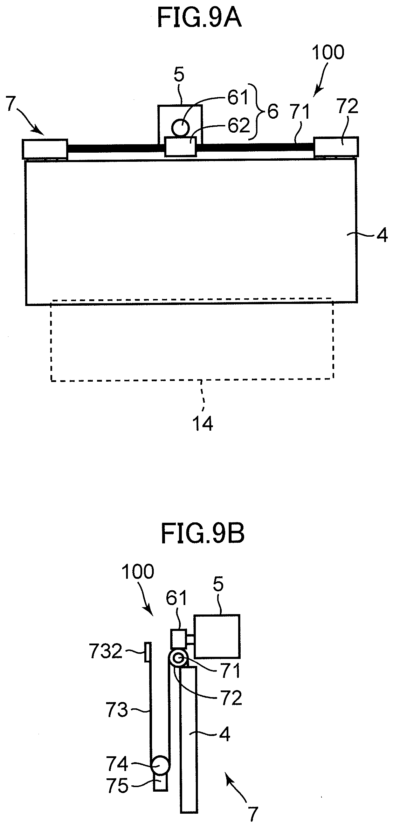

Next, operation of the automatic door device 100 will be described with reference to FIGS. 8A to 9B. FIG. 8A is a front view when the opening/closing door 4 is in the closed state, and FIG. 8B is a side view thereof. FIG. 9A is a front view when the opening/closing door 4 is in the open state, and FIG. 9B is a side view thereof.

In the state shown in FIGS. 8A and 8B, the opening/closing door 4 covers the opening 14 and closes the opening 14. The moving pulley 74 of the mechanical mechanism 7 is in the most elevated position. The dog 81 (FIGS. 3 and 4) mounted on the weight unit 75 interlocked with the moving pulley 74 causes the sensor 8 to operate, and the sensor 8 detects the closing of the opening/closing door 4. As described above, in the magnetic coupling part 6, the first magnetic coupling member 61 and the second magnetic coupling member 62 are magnetically coupled to each other, and the driving force can be transmitted.

When an opening instruction for the opening/closing door 4 is given from the state of FIGS. 8A and 8B, the motor device 5 operates. When the motor shaft 51 is driven, the first magnetic coupling member 61 also rotates. When the first magnetic coupling member 61 rotates, the second magnetic coupling member 62 also rotates on the basis of the magnetic coupling force, and as a result, the shaft 71 also rotates about the axis. That is, the rotational driving force of the motor shaft 51 is transmitted to the shaft 71 through the magnetic coupling part 6. As a result of the rotation of the shaft 71, the belt gears 72 located at the opposite ends of the shaft 71 also rotate. The belt 73 engaged with the belt gear 72 moves according to the rotation of the belt gear 72. The opening/closing door 4 moves in the up-down direction according to the movement of the belt 73. In the example of FIGS. 8A and 8B, the belt 73 moves so that the opening/closing door 4 is lifted upward. A rotation direction of the motor shaft 51 is selected so that such movement of the belt 73 is performed.

FIGS. 9A and 9B illustrate a state where lifting of the opening/closing door 4 is completed. The opening/closing door 4 is lifted to above the opening 14, so that the opening 14 is opened toward the outside. The moving pulley 74 is suspended most downward. In the present embodiment, since the weight of the weight unit 75 acts, a lifting load of the opening/closing door 4 can be reduced. That is, the opening/closing door 4 can be operated with a small driving force. Therefore, a magnetic coupling force of the magnetic coupling part 6 can be set relatively small, and the magnetic coupling members 61 and 62 can be downsized. In addition, a load on the motor device 5 can be reduced, so that a small motor device can be adopted. Furthermore, the weight unit 75 is integrated with the moving pulley 74. Therefore, as compared with a case where a weight member is simply attached to an end part of the belt 73, a movement amount of the weight unit 75 in the up-down direction can be reduced to about 1/2, which can contribute to the miniaturization of the automatic door device 100.

When the opening/closing door 4 is closed from the state of FIGS. 9A and 9B, the motor shaft 51 is reversely driven. A driving force of the motor shaft 51 is transmitted to the shaft 71 through the magnetic coupling part 6 to move the belt 73 in the opposite direction. That is, the belt 73 is moved in a direction in which the opening/closing door 4 is lowered. The opening/closing door 4 continues to lower until closing the opening 14. When the sensor 8 eventually detects the closing of the opening/closing door 4, i.e., when returning to the state of FIG. 8, the driving of the motor shaft 51 is stopped.

When a phenomenon that hinders the opening and closing operation of the opening/closing door 4 due to the operation of the motor device 5 occurs, the magnetic coupling part 6 blocks transmission of a motor torque to the opening/closing door 4. For example, it is assumed that a foreign matter or an object such as a finger of the worker is caught between a lower edge part of the opening 14 and a lower edge of the opening/closing door 4 while the opening/closing door 4 is lowering. In this case, since the lowering of the opening/closing door 4 is hindered by the object, even when the motor shaft 51 and the first magnetic coupling member 61 rotate, the second magnetic coupling member cannot rotate. That is, in the magnetic coupling part 6, the driving force transmission by the magnetic coupling force is not performed, and only the first magnetic coupling member 61 idles. Therefore, a lowering force does not act on the opening/closing door 4.

In a case where the driving force is transmitted not by the magnetic coupling part 6 but by a mechanical method such as gear meshing, even when catching of an object by the opening/closing door 4 or the like occurs, the meshing is not released, so that the lowering force continues to act on the opening/closing door 4. The driving force of the motor shaft 51 continues to be applied to the caught object via the opening/closing door 4. When a finger is caught, it can be dangerous. Therefore, it is necessary to provide a sensor that detects abnormal operation of the opening/closing door 4, a mechanism that automatically reversely drives the motor shaft 51 at the time of abnormality detection, and the like. By contrast, according to the present embodiment, when an excessive force acts on the opening/closing door 4 due to a factor such as catching of an object, the magnetic coupling of the magnetic coupling part 6 is released. Therefore, the driving force of the motor shaft 51 is not continuously applied to the object caught by the opening/closing door 4, and a mechanism for automatically reversing the opening/closing door 4 is also unnecessary. In addition, minimum sensors for monitoring the operation of the opening/closing door 4 need to be installed. Therefore, a safety mechanism for the opening/closing door 4 can be simplified.

FIGS. 8A to 9B illustrate the example in which the opening 14 is opened by the rising of the opening/closing door 4 from the state in which the opening/closing door 4 closes the opening 14. Instead of this mode, the opening 14 may be opened by the lowering of the opening/closing door 4 from the state of FIGS. 8A and 8B. Alternatively, the automatic door device 100 may be disposed in the housing 10 such that the shaft 71 is positioned near an upper edge of the opening 14, so that FIGS. 8A and 8B may illustrate a state in which the opening 14 is opened by the opening/closing door 4, and FIGS. 9A and 9B may illustrate a state in which the opening 14 is closed by the opening/closing door 4.

### [Modification]

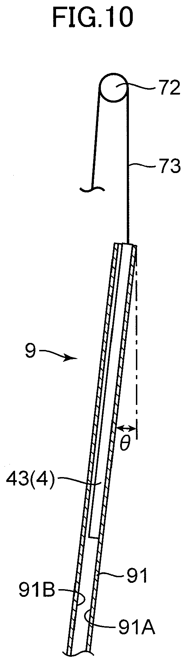

FIG. 10 is a cross-sectional view illustrating a modification of the opening/closing door 4 and the guide member 9 thereof. Although the resin rail 91 of the guide member 9 may be disposed so as to extend in the vertical direction, it may be disposed so as to extend in a direction inclined at a predetermined angle θ with respect to the vertical direction as illustrated in FIG. 10. According to this modification, the opening/closing door 4 is guided by the inclined resin rail 91, so that the opening/closing door 4 is also inclined by a predetermined angle θ. Therefore, a sealability between the opening/closing door 4 and the housing 10 can be secured by a weight of the opening/closing door 4. On an inner surface of the U-shaped cross section, the resin rail 91 has an inner surface 91A positioned on an inner side of the inclination and an outer surface 91B positioned on an outer side. When the opening/closing door 4 is inclined, the side end edge 43 of the opening/closing door 4 naturally comes into close contact with the inner surface 91A by its own weight. Due to this close contact, the sealability can be enhanced.

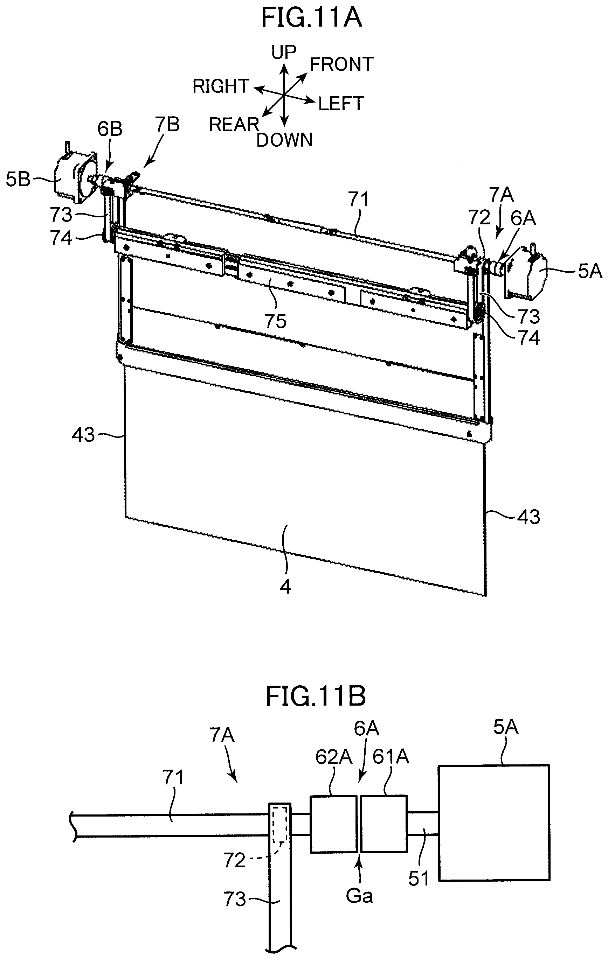

FIG. 11A is a perspective view of an automatic door device 100A according to the modification, and FIG. 11B is a schematic view of a magnetic coupling part 6A included in the automatic door device 100A. The automatic door device 100A includes, as a motor device, a first motor 5A including the first motor shaft 51 and a second motor 5B including a second motor shaft. The first motor 5A is disposed on the left end side of the shaft 71, the second motor 5B is disposed on the right end side of the shaft 71, and the motors 5A and 5B are opposed to each other across the shaft 71. That is, the motor shaft 51 and the shaft 71 are disposed coaxially.

A first magnetic coupling member 61A of the magnetic coupling part 6A is attached to the first motor shaft 51. A second magnetic coupling member 62A is attached to the right end of the shaft 71. An axis of the first magnetic coupling member 61A and an axis of the second magnetic coupling member 62A are coaxial, and the two are opposed to each other with the gap Ga interposed therebetween. When the first magnetic coupling member 61A rotates by rotation of the first motor shaft 51, the second magnetic coupling member 62A also rotates by a magnetic coupling force. As a result, the shaft 71 also rotates. The second motor 5B is also provided with a magnetic coupling part 6B having a similar mode.

A driving force of the first motor 5A is transmitted to the side end edge 43 (a first position) on the left side of the opening/closing door 4 through the belt gear 72 and the belt 73 on the left side (a first driving force transmission path). A driving force of the second motor 5B is transmitted to the side end edge 43 (a second position) on the right side of the opening/closing door 4 through the belt gear 72 and the belt 73 on the right side (a second driving force transmission path). At the time of opening/closing of the opening/closing door 4, the first motor 5A and the second motor 5B are operated synchronously. According to the automatic door device 100A, the opening/closing door is operated using the two motors, the two sets of coupling members, and the two driving force transmission paths. Since a load of each of the motors 5A and 5B is reduced, an opening and closing mechanism of the opening/closing door 4 can be constructed using an inexpensive small motor.

As a modification of the automatic door device 100A, the opening/closing door 4 may be divided into left and right pieces, and the left divided piece may be operated by the first motor 5A and the right divided piece may be operated by the second motor 5B. In this case, the second motor 5B may be omitted, the magnetic coupling part may be provided in the middle of the shaft 71, and both the left divided piece and the right divided piece of the opening/closing door 4 may be operated by the driving force of the first motor SA.

### [Inventions Included in Above Embodiments]

The embodiments described above include the following inventions.

A work device according to one aspect of the present invention includes: a housing; a work unit that is housed in the housing and performs predetermined work on an object disposed inside the housing; an opening/closing door attached to the housing; a motor device including a motor shaft that outputs a driving force for operating the opening/closing door; a first magnetic coupling member attached to the motor shaft; a second magnetic coupling member magnetically coupled to the first magnetic coupling member; and a mechanical mechanism that mechanically connects the second magnetic coupling member and the opening/closing door, and transmits, to the opening/closing door, a driving force of the motor shaft applied from the first magnetic coupling member to the second magnetic coupling member by the coupling.

According to this mode, a magnetic coupling part between the first magnetic coupling member and the second magnetic coupling member exists in a driving force transmission path from the motor shaft of the motor device to the opening/closing door. When an excessive force acts on the opening/closing door due to a factor such as catching of a foreign substance, the magnetic coupling part is released. Therefore, the driving force of the motor shaft is not continuously applied to the object caught by the opening/closing door, and a mechanism for reversely operating the opening/closing door becomes unnecessary. In addition, minimum sensors for monitoring the operation of the opening/closing door need to be installed. Therefore, a safety mechanism for the opening/closing door can be simplified.

In the work device described above, the housing may be a housing for a cell moving device in which a first container serving as a movement source of a cell and a second container serving as a movement destination of a cell can be disposed, and the work unit may be a head unit that picks a cell from the first container and moves the cell to the second container.

According to this mode, by closing the opening/closing door during the operation of the head unit, worker's access to the inside of the housing can be restricted. In addition, a safety mechanism for the opening/closing door attached to the housing for the cell moving device can be simplified.

The work device described above may have a configuration in which the mechanical mechanism includes a belt that suspends and supports the opening/closing door, the housing includes an opening that enables access to the inside of the housing, and the opening/closing door moves in an up-down direction with respect to the opening by movement of the belt by the driving force of the motor shaft.

According to this mode, movement of the belt that suspends and supports the opening/closing door causes the opening/closing door to move up and down. By adopting a mechanical mechanism using such a belt, it is possible to simplify a structure for moving the opening/closing door up and down as compared with a case where a ball screw shaft or the like is used.

The work device described above may have a configuration in which the work device further includes a plurality of belts as the belt, in which the mechanical mechanism further includes a shaft to which the second magnetic coupling member is attached, the plurality of belts are engaged with the shaft, and the shaft rotates about an axis and the plurality of belts are moved by the rotation of the shaft when a rotational driving force of the motor shaft is applied from the first magnetic coupling member to the second magnetic coupling member.

According to this mode, since the opening/closing door is suspended and supported by the plurality of belts engaged with the shaft, the opening/closing door can be stably operated. The driving force is transmitted to the opening/closing door just by rotating the shaft about the axis. Therefore, the structure of the mechanical mechanism can be simplified.

In the work device described above, it is desirable that the opening/closing door has a rectangular shape in plan view, and the plurality of belts are attached to one end and the other end of the opening/closing door in a width direction.

According to this mode, it is possible to stably support the rectangular opening/closing door and to stably move the opening/closing door up and down.

In the work device described above, it is desirable that the plurality of belts include a first belt engaged with one end of the shaft and a second belt engaged with the other end of the shaft, and the second magnetic coupling member is attached to an intermediate position in an axial direction of the shaft.

According to this mode, the first belt and the second belt are respectively engaged with the opposite ends of the shaft, and the second magnetic coupling member to which the driving force is applied is disposed at the center of the shaft. Therefore, well-balanced power transmission can be realized.

In the work device described above, the mechanical mechanism may further include: a shaft to which the second magnetic coupling member and an engaging wheel are attached; and a moving pulley to which a weight member is attached, an upper end side of the belt may be stretched around the engaging wheel and the moving pulley so as to be folded back, and a lower end side of the belt may suspend and support the opening/closing door, and the shaft may rotate about an axis and the plurality of belts may be moved by driving of the engaging wheel accompanying the rotation when a rotational driving force of the motor shaft is applied from the first magnetic coupling member to the second magnetic coupling member.

According to this mode, since the moving pulley to which the weight member is attached is used, the opening/closing door can be operated with a small driving force. Therefore, a magnetic coupling force can be set relatively small, and the first magnetic coupling member and the second magnetic coupling member can be downsized. In addition, a load on the motor device can be reduced, so that a small motor device can be adopted.

In the work device described above, it is desirable that the mechanical mechanism further includes a shaft to which the second magnetic coupling member is attached, and the motor shaft and the shaft are disposed orthogonal to each other.

In a case where the motor shaft and the shaft are disposed in the same direction, since the motor device is generally larger in size than the shaft, a housability of the motor device in the housing is deteriorated. That is, in order to house a part where the motor device protrudes more than the shaft, it is necessary to make the housing large or irregular. According to the above mode, since the motor shaft and the shaft are disposed to be orthogonal to each other, the housability of the motor device in the housing can be improved.

The work device described above desirably further includes a sensor that detects an open/closed state of the opening/closing door. According to this mode, it is possible to perform various control, notification, and the like by detecting the open/closed state of the opening/closing door.

In the work device described above, it is desirable that the opening/closing door has a rectangular shape in plan view, and the housing includes a guide member that is disposed at a position corresponding to a side of the opening/closing door and guides movement of the opening/closing door in an up-down direction.

According to this mode, since the opening/closing door is guided by the guide member when moving in the up-down direction, opening and closing operation of the opening/closing door can be stabilized.

In the above work device, at least a part of the guide member in contact with the opening/closing door is preferably made of resin because an operation resistance of the opening/closing door can be reduced.

In the work device described above, the guide member may extend in a direction inclined with respect to a vertical direction.

According to this mode, the opening/closing door has an attitude inclined by the guide member. Therefore, a sealability between the opening/closing door and the housing can be secured by a weight of the opening/closing door.

In the work device described above, the motor device may include a first motor including a first motor shaft and a second motor including a second motor shaft, the first magnetic coupling member may be attached to each of the first motor shaft and the second motor shaft, and the second magnetic coupling member may be magnetically coupled to each corresponding first magnetic coupling member, and the mechanical mechanism may transmit the driving force by a first driving force transmission path from the first motor shaft to a first position of the opening/closing door and a second driving force transmission path from the second motor shaft to a second position of the opening/closing door.

According to this mode, the opening/closing door is operated using the two motors, the two sets of coupling members, and the two driving force transmission paths. Since a load of an individual motor is reduced, an opening and closing mechanism of the opening/closing door can be constructed using an inexpensive small motor.

According to the present invention described above, it is possible to provide a work device having an opening/closing door and enabling avoidance of complication of a structure while securing safety.

## Claims

1. A work device comprising:
a housing;
a work unit that is housed in the housing and performs predetermined work on an object disposed inside the housing;
an opening/closing door attached to the housing;
a motor device including a motor shaft that outputs a driving force for operating the opening/closing door;
a first magnetic coupling member attached to the motor shaft;
a second magnetic coupling member magnetically coupled to the first magnetic coupling member; and
a mechanical mechanism that mechanically connects the second magnetic coupling member and the opening/closing door, and transmits, to the opening/closing door, a driving force of the motor shaft applied from the first magnetic coupling member to the second magnetic coupling member by the coupling.

2. The work device according to claim 1, wherein
the housing is a housing for a cell moving device in which a first container serving as a movement source of a cell and a second container serving as a movement destination of a cell can be disposed, and
the work unit includes a head unit that picks a cell from the first container and moves the cell to the second container.

3. The work device according to claim 1 or 2, wherein
the mechanical mechanism includes a belt that suspends and supports the opening/closing door,
the housing includes an opening that enables access to the inside of the housing, and
the opening/closing door moves in an up-down direction with respect to the opening by movement of the belt by the driving force of the motor shaft.

4. The work device according to claim 3, further comprising a plurality of belts as the belt,
wherein the mechanical mechanism further includes a shaft to which the second magnetic coupling member is attached,
the plurality of belts are engaged with the shaft, and
the shaft rotates about an axis and the plurality of belts are moved by the rotation of the shaft when a rotational driving force of the motor shaft is applied from the first magnetic coupling member to the second magnetic coupling member.

5. The work device according to claim 4, wherein
the opening/closing door has a rectangular shape in plan view, and
the plurality of belts are attached to one end and the other end of the opening/closing door in a width direction.

6. The work device according to claim 4, wherein
the plurality of belts include a first belt engaged with one end of the shaft and a second belt engaged with the other end of the shaft, and
the second magnetic coupling member is attached to an intermediate position in an axial direction of the shaft.

7. The work device according to claim 3, wherein
the mechanical mechanism further includes a shaft to which the second magnetic coupling member and an engaging wheel are attached, and a moving pulley to which a weight member is attached,
an upper end side of the belt is stretched around the engaging wheel and the moving pulley so as to be folded back, and a lower end side of the belt suspends and supports the opening/closing door, and
the shaft rotates about an axis and the plurality of belts are moved by driving of the engaging wheel accompanying the rotation when a rotational driving force of the motor shaft is applied from the first magnetic coupling member to the second magnetic coupling member.

8. The work device according to claim 1 or 2, wherein
the mechanical mechanism further includes a shaft to which the second magnetic coupling member is attached, and
the motor shaft and the shaft are disposed orthogonal to each other.

9. The work device according to claim 1 or 2, further comprising a sensor that detects an open/closed state of the opening/closing door.

10. The work device according to claim 1 or 2, wherein
the opening/closing door has a rectangular shape in plan view, and
the housing includes a guide member that is disposed at a position corresponding to a side of the opening/closing door and guides movement of the opening/closing door in an up-down direction.

11. The work device according to claim 10, wherein at least a part of the guide member in contact with the opening/closing door is made of resin.

12. The work device according to claim 10, wherein the guide member extends in a direction inclined with respect to a vertical direction.

13. The work device according to claim 1 or 2, wherein
the motor device includes a first motor including a first motor shaft and a second motor including a second motor shaft,
the first magnetic coupling member is attached to each of the first motor shaft and the second motor shaft, and the second magnetic coupling member is magnetically coupled to each corresponding first magnetic coupling member, and
the mechanical mechanism transmits the driving force by a first driving force transmission path from the first motor shaft to a first position of the opening/closing door and a second driving force transmission path from the second motor shaft to a second position of the opening/closing door.
